# EUROPEAN PATENT APPLICATION

(11) **EP 2 752 818 A2**
(43) Date of publication of application: **09.07.2014**
(21) Application number: 13166734.7
(22) Date of filing: 07.05.2013
(51) Int. Cl.: G06T 15/08, G06T 15/50

(54) **Method and apparatus for providing medical images**

(30) Priority: 04.01.2013 KR 20130001211
(71) Applicant: Samsung Medison Co., Ltd., Gangwon-do 250-870 (KR)
(72) Inventor: Yoo, Jun-sang, Hongcheon-gun, Gangwon-do (KR); Oh, Dong-hoon, Hongcheon-gun, Gangwon-do (KR); Kim, Sung-yun, Hongcheon-gun, Gangwon-do (KR)
(74) Representative: Lorenz, Markus

(57) **Abstract**

A method and an apparatus for providing a medical image from volume data for an object are provided. The method includes: determining characteristics of a light source that radiates light onto the object which is located in a virtual space, based on a received user input; generating a three-dimensional (3D) image by performing a rendering on the volume data, based on the determined characteristics of the light source; and displaying the generated 3D image.

## Description

### CROSS-REFERENCE TO RELATED PATENT APPLICATION

This application claims the benefit of Korean Patent Application No. 10-2013-0001211, filed on January 4, 2013, in the Korean Intellectual Property Office, the disclosure of which is incorporated herein in its entirety by reference.

### BACKGROUND OF THE INVENTION

### 1. Field of the Invention

The present invention relates to a method and apparatus for providing a medical image, and more particularly, to a method and apparatus for providing a three-dimensional (3D) medical image by rendering ultrasonic volume data for an object.

### 2. Description of the Related Art

An ultrasonic system has non-invasive and non-destructive characteristics and thus is widely used in various medical fields to obtain information regarding the inside of an object, i.e., a living body. Since the ultrasonic system may provide a high resolution image of the inside of the object to doctors in real-time without the need for surgery through which the object is incised to observe the inside of the object, the use of the ultrasonic system is important in the medical field.

A recent ultrasonic system provides a three-dimensional (3D) ultrasonic image including clinical information such as spatial information and anatomical shapes that cannot be provided by a two-dimensional (2D) ultrasonic image. That is, the recent ultrasonic system transmits an ultrasonic signal to an object, receives an ultrasonic signal (an ultrasonic echo signal) that is reflected from the object, and forms volume data based on the received ultrasonic signal. In addition, the recent ultrasonic system forms a 3D ultrasonic image including clinical information by rendering the formed volume data.

'volume rendering' is a technology for displaying a 2D projection image of a 3D discretely sampling data set, such as volume data. An example of a method of rendering volume data includes a ray casting method by which a virtual ray is cast to an object located in a virtual space and a reflected light is calculated.

### SUMMARY OF THE INVENTION

The present invention provides a method and apparatus for providing a more realistic and detailed three-dimensional (3D) image with respect to a region of interest for an object by performing a volume rendering for volume data of the object based on characteristics of an adjustable light source.

According to an aspect of the present invention, there is provided a method of providing a medical image from volume data for an object, the method including: determining characteristics of a light source that radiates light onto the object which is located in a virtual space, based on a received user input; generating a three-dimensional (3D) image by performing a rendering on the volume data, based on the determined characteristics of the light source; and displaying the generated 3D image.

The characteristics of the light source may include at least one of a distance from the object to the light source, a radiation angle of the light source, and a radiation direction of the light source.

The determining of the characteristics of the light source may include determining a distance from the object to the light source, and the generating of the 3D image may include adjusting a brightness of at least a partial area of the 3D image based on the determined distance from the object to the light source.

The determining of the characteristics of the light source may include determining a radiation angle of the light source, and the generating of the 3D image may include adjusting the extent of a shadow spread of at least a partial area of the 3D image based on the determined radiation angle of the light source.

The determined characteristics of the light source may include characteristics of a spot light or a point light.

The generating of the 3D image may include: casting a virtual ray to the volume data; shading sampling points on the virtual ray based on the determined characteristics of the light source; and calculating a final color value of a predetermined pixel included in the 3D image from a cumulative value of shading values of the sampling points.

The method may further include: generating a directional light image by performing a rendering on the volume data based on characteristics of a directional light; and displaying the generated directional light image, wherein the displaying of the generated 3D image includes replacing the directional light image with the 3D image and then displaying the 3D image, based on the user input.

The generating of the 3D image may include: casting a virtual ray to the volume data; shading sampling points on the virtual ray based on characteristics of a directional light passing through a predetermined filter; and calculating a final color value of a predetermined pixel included in the 3D image from a cumulative value of shading values of the sampling points, wherein the predetermined filter corresponds to the determined characteristics of the light source.

The generating of the 3D image may include: generating a directional light image by performing a rendering on the volume data based on characteristics of a directional light; and transforming the directional light image based on the determined characteristics of the light source.

The determining of the characteristics of the light source may include determining at least one of a shape, size, and gradation effect of at least a partial surface of the object that is brightly shown by radiating light thereto from the light source.

According to another aspect of the present invention, there is provided an apparatus for providing a medical image, the apparatus including: a data acquisition unit that acquires volume data for an object; a receiver that receives a user input; an image generation unit that determines characteristics of a light source radiating light onto the object located in a virtual space based on the received user input and generates a 3D image by performing a rendering on the volume data based on the determined characteristics of the light source; and a display unit that displays the generated 3D image.

The characteristics of the light source may include at least one of a distance from the object to the light source, a radiation angle of the light source, and a radiation direction of the light source.

The image generation unit may determine a distance from the object to the light source as the characteristics of the light source, and may adjust a brightness of at least a partial area of the 3D image based on the determined distance from the object to the light source.

The image generation unit may determine a radiation angle of the light source as the characteristics of the light source, and may adjust the extent of a shadow spread of at least a partial area of the 3D image based on the determined radiation angle of the light source.

The image generation unit may determine the characteristics of the light source by determining at least one of a shape, size, and gradation effect of at least a partial surface of the object that is brightly shown by radiating light thereto from the light source.

According to another aspect of the present invention, there is provided a computer-readable recording medium having embodied thereon a computer program for executing the method.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram of an apparatus for providing a medical image, according to an embodiment of the present invention;
FIG. 2 is a flowchart illustrating a method of providing a medical image, according to an embodiment of the present invention;
FIGS. 3A and 3B are diagrams for explaining the apparatus for providing a medical image, according to the embodiment of the present invention, which generates a three-dimensional (3D) image based on characteristics of a light source;
FIG. 4A is a diagram for explaining a directional light;
FIG. 4B is a diagram for explaining a point light;
FIG. 4C is a diagram for explaining a spot light;
FIG. 5A illustrates an example of a 3D image that is displayed according to a feto-realistic view (FRV);
FIG. 5B illustrates an example of a 3D image that is displayed in the apparatus for providing a medical image according to the embodiment of the present invention;
FIGS. 6A through 6C are diagrams for explaining the apparatus of generating a 3D image according to an the embodiment of the present invention, which generates a 3D image based on characteristics of a light source;
FIG. 7A illustrates 3D images that are displayed according to an FRV; and
FIG. 7B illustrates 3D images that are displayed in the apparatus of generating a 3D image according to the embodiment of the present invention.

### DETAILED DESCRIPTION OF THE INVENTION

As used herein, expressions such as "at least one of," when preceding a list of elements, modify the entire list of elements and do not modify the individual elements of the list.

The present invention is described more fully hereinafter with reference to the accompanying drawings, in which embodiments of the invention are shown.

While terms used in the present invention are selected, wherever possible, from terms currently in widespread use, the terms may vary according to an operator's intention, judicial precedents, or the emergence of new technology. In exceptional cases, terms arbitrarily selected by the applicants may be used. In these cases, the meanings of the terms should be understood in consideration not of simple names of the terms but of meanings described or used in the detailed description of the present invention.

It will be further understood that the terms "comprises", "comprising", "includes", and/or "including" when used herein, specify the presence of stated features, items, steps, operations, elements, and/or components, but do not preclude the presence or addition of one or more other features, items, steps, operations, elements, components, and/or groups thereof. Also, the term "unit" refers to a unit configured to process at least one function or operation and may be embodied by hardware, software, or a combination of hardware and software.

In this specification, the term "object" may refer to a portion of the human body. For example, the object may include internal organs, such as the liver, the heart, the womb, the brain, the breast, and the abdomen, or fetuses. In addition, the object may include a cross-section of a portion of the human body. In this specification, the term "user" may refer to a medical expert, such as a doctor, a nurse, a clinical pathologist, a medical image expert, or a patient, but it is not limited thereto.

In addition, in this specification, the expression "three-dimensional (3D) image" refers to an image obtained by indicating a space sense on a two-dimensional (2D) plane through the contrast, color, size, and position of an object. The 3D image includes both a still image and a video image. Examples of a technology for generating the 3D image may include any 3D imaging technology that may be implemented in the future, as well as commercialized 3D imaging technologies.

The present invention relates to a method of restoring a 3D image from volume data obtained from an object. Thus, the present invention may be widely applied to a medical imaging method of restoring a 3D image of a human anatomy from signals projected on the human anatomy, such as magnetic resonance imaging (MRI), computerized tomography (CT), and positron emission tomography (PET), as well as an ultrasonic diagnostic method.

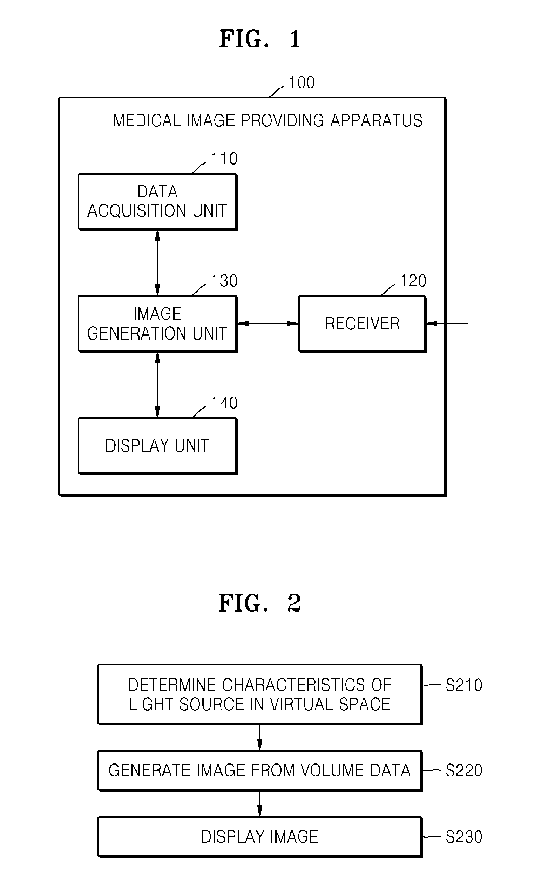

FIG. 1 is a block diagram of an apparatus 100 for providing a medical image (hereinafter, referred to as a medical image providing apparatus), according to an embodiment of the present invention.

As illustrated in FIG. 1, the medical image providing apparatus 100 may include a data acquisition unit 110, a receiver 120, an image generation unit 130, and a display unit 140. However, the present invention is not limited thereto. That is, the medical image providing apparatus 100 may be implemented with more components than those illustrated in FIG. 1 or may be implemented with less components than those illustrated in FIG. 1.

The data acquisition unit 110 may acquire volume data for an object. The "volume data" may refer to data including clinical information, such as spatial information and anatomical shapes of an object. For example, an ultrasonic system may transmit an ultrasonic signal to an object, receive an ultrasonic signal (namely, an ultrasonic echo signal) that is reflected from the object, and form volume data by using the received ultrasonic echo signal. In addition, a recent ultrasonic system forms a 3D ultrasonic image including clinical information by rendering the formed volume data. Thus, the data acquisition unit 110 may include a probe (not shown) for transmitting or receiving an ultrasonic signal and a beamformer (not shown) for performing transmission focusing and reception focusing of the ultrasonic signal. In addition, the data acquisition unit 110 may acquire volume data of an object from a memory (not shown) in which the volume data is previously stored.

The receiver 120 may receive a user input for characteristics of a light source that radiates light onto an object that is located in a virtual space.

In addition, the receiver 120 may include an input unit through which a user inputs data for controlling the medical image providing apparatus 100. Examples of the receiver 120 may include a key pad, a dome switch, a touch pad (a contact capacitance type, a pressure resistance type, an infrared sensing type, a surface ultrasonic wave conduction type, an integral tension measurement type, a piezoelectric effect type, etc.), a jog wheel, a jog switch, etc. However, the present invention is not limited thereto.

The image generation unit 130 may determine the characteristics of the light source that radiates light onto the object that is located in the virtual space, based on the user input received by the receiver 120. The image generation unit 130 may generate a 3D image by performing a rendering on the volume data, based on the determined characteristics of the light source.

The display unit 140 may display the 3D image generated by the image generation unit 130. In addition, the display unit 130 may display a user interface (UI) or graphic user interface (GUI) related to setting functions.

The medical image providing apparatus 100 may further include a controller (not shown). The controller generally controls the overall operation of the medical image providing apparatus 100. For example, the controller may control the data acquisition unit 110, the receiver 120, the image generation unit 130, and the display unit 140 by executing programs stored in a memory (not shown).

Hereafter, a method by which the medical image providing apparatus 100 displays a medical image by using the components described above is described in detail with reference to FIG. 2.

FIG. 2 is a flowchart illustrating a method of providing a medical image, according to an embodiment of the present invention.

Referring to FIG. 2, the method of providing a medical image may include operations that are processed in the medical image providing apparatus 100 illustrated in FIG. 1. Thus, although omitted below, the descriptions provided above of the medical image providing apparatus 100 illustrated in FIG. 1 apply to the method of FIG. 2.

In Operation S21 0, the medical image providing apparatus 100 may receive a user input and may determine characteristics of a light source based on the user input. The light source may include a virtual light source for radiating light onto an object located in a virtual space so that the medical image providing apparatus 100 may generate a 3D image from volume data for the object.

The medical image providing apparatus 100 may determine characteristics of a light source by defining an effect of the light source on an initial face of the volume data, which is shown in a direction to which light is radiated. The medical image providing apparatus 100 may define a light source and determine characteristics of the light source by determining the shape, the size and the gradation effect of reflected light, which apply to the initial face of the volume data. That is, the medical image providing apparatus 100 may determine the characteristics of the light source by defining at least one of a shape, a size, and a gradation effect of the surface of an object that is brightly shown by radiating light thereto from the light source, based on a user input.

The characteristics of the light source, which are determined by the medical image providing apparatus 100, may include at least one of a distance from an object in the virtual space to the light source, a radiation angle of the light source, and a radiation direction of the light source. The radiation angle of the light source may be a radiation angle of a light source that radiates light in a conical shape, for example, an angle of a light cone that is formed by a spotlight. The surface area that the light source lights widens when the radiation angle of the light source increases.

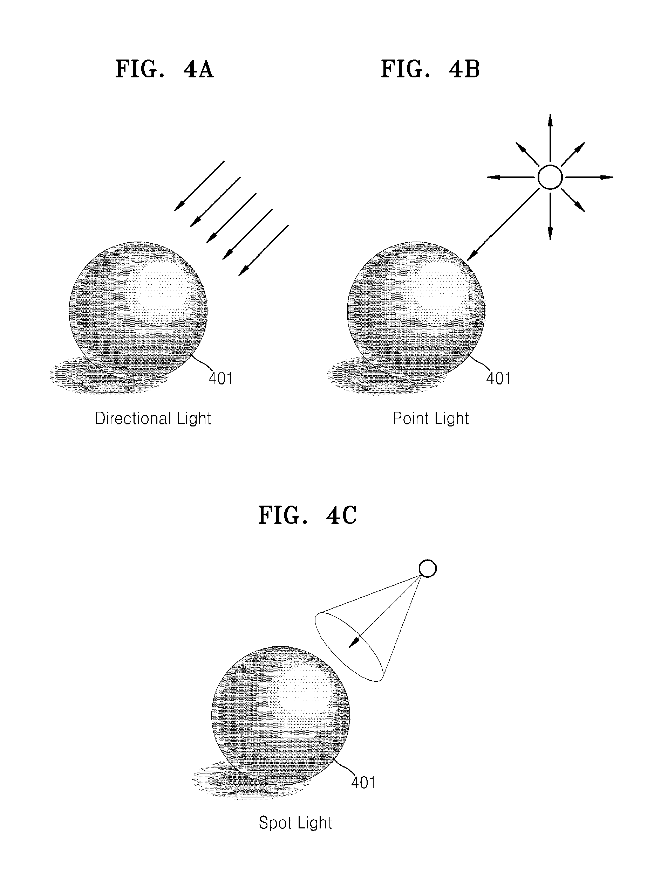

The characteristics of the light source, which are determined by the medical image providing apparatus 100, may include characteristics of a spot light or point light. The characteristics of the light source are described in detail with reference to FIG. 4 below.

In Operation S220, the medical image providing apparatus 100 may generate a 3D image by performing a rendering on the volume data for the object, based on the characteristics of the light source, which are determined in Operation S210.

For example, a "ray casting" method may be used to render the volume data. In the ray casting method, a virtual light ray is cast onto a voxel. A "voxel" indicates a minute three-dimensional structural unit of an object. The volume data of the object may be formed of a plurality of voxels.

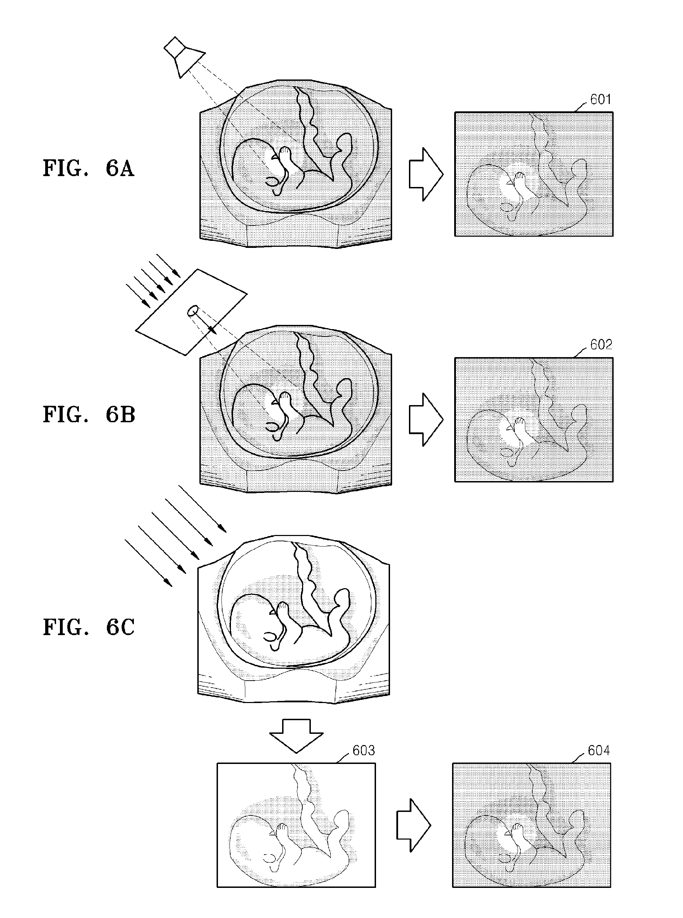

The medical image providing apparatus 100 calculates a reflected light of each of the voxels to which light reaches, and obtains a pixel value of each pixel included in a 3D image. The pixel value may include color information, e.g., RGB values. A method of generating a 3D image is described in detail with reference to FIG. 6 below.

The medical image providing apparatus 100 may generate a 3D image based on a distance from an object to the light source in the virtual space, which is determined in Operation S210. In this case, the medical image providing apparatus 100 may adjust the brightness of at least a partial area of the object represented in the 3D image, according to the determined distance. The at least a partial area of which the brightness is adjusted may comprise an area that the light source lighted.

The medical image providing apparatus 100 may generate the 3D image based on the radiation angle of the light source, which is determined in Operation S210. In this case, the medical image providing apparatus 100 may adjust the shadow spread of at least a partial area of the object in the 3D image, on which light is radiated from the light source, according to the determined radiation angle.

In Operation S230, the medical image providing apparatus 100 may display the 3D image generated in Operation S220.

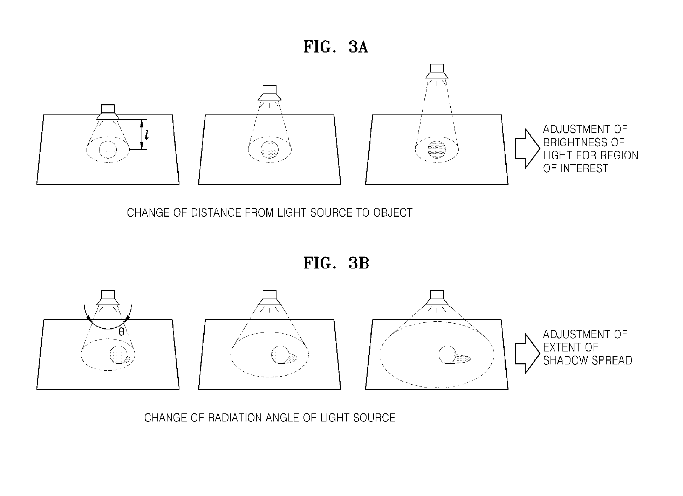

The characteristics of the light source, which are determined in the medical image providing apparatus 100, are described with reference to FIGS. 3 and 4 below.

FIGS. 3A and 3B are diagrams for explaining the medical image providing apparatus 100 that generates a 3D image based on characteristics of a light source.

As illustrated in FIG. 3A, the brightness of at least a partial area of a 3D image is adjusted when adjusting a distance between an object and the light source in a virtual space. For example, when the light source radiating light onto the object is located close to the object, a 3D image in which at least a partial area of the object is more brightly represented is generated.

In addition, as illustrated in FIG. 3B, the shadow spread of at least a partial area of a 3D image is adjusted when adjusting a radiation angle Θ of the light source. For example, when the radiation angle of the light source radiating light onto the object that is located in the virtual space is relatively large, a 3D image in which a shadow of the object is represented widely spread is generated. The larger the extent of the shadow spread is, the more the edge of the shadow that is shown in the 3D image is smoothly represented.

Thus, the medical image providing apparatus 100 provides a more realistic and detailed 3D image with respect to a region of interest of the object, e.g., a fetal wrinkle or cleft lip, by determining the brightness of the object or the extent of the shadow spread in the 3D image based on a user input, and thus improves a user's concentration on the 3D image.

FIG. 4A is a diagram for explaining a directional light, FIG. 4B is a diagram for explaining a point light, and FIG. 4C is a diagram for explaining a spot light.

FIG. 4A illustrates an object 401 onto which the directional light is radiated in a virtual space. As illustrated in FIG. 4A, a light source of the directional light is not located in the virtual space, and applies the same amount of light into the virtual space at a specific angle. Thus, when a 3D image of an object is generated based on characteristics of the directional light, it is possible to obtain the same effect as when the light of the sun is radiated onto the object.

FIG. 4B illustrates the object 401 onto which the point light is radiated in a virtual space. As illustrated in FIG. 4B, the point light is located at a specific position in the virtual space and radiates light in all directions. Thus, when a 3D image of an object is generated based on characteristics of the point light, it is possible to obtain the same effect as when a light bulb lights the object.

FIG. 4C illustrates the object 401 onto which the spot light is radiated in a virtual space. As illustrated in FIG. 4C, the spot light is located at a specific position in the virtual space, and radiates light into a portion of the virtual space at a specific angle or an angle within a predetermined range. Thus, when a 3D image of an object is generated based on characteristics of the spot light, it is possible to obtain the same effect as when a flashlight lights the object.

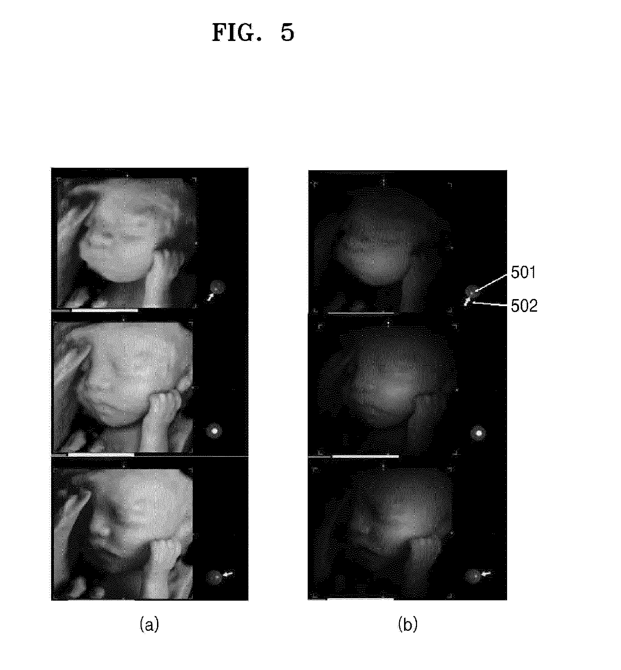

FIG. 5A illustrates an example of a 3D image that is displayed according to a feto-realistic view (FRV). The FRV is one of the methods of indicating a 3D ultrasonic image by using a translucent volume rendering method. The 3D image of a fetus that is displayed according to the FRV is generated based on a directional light. Thus, although FIG. 5A illustrates an image of a fetus in a mother's womb, a user feels as if the user is looking at an image of a fetus exposed to the outside where the sun shines.

FIG. 5B illustrates an example of a 3D image that is displayed in the medical image providing apparatus 100 according to an embodiment of the present invention.

Compared to FIG. 5A, in FIG. 5B, an image showing a fetus, to whom a spot light instead of the directional light is lit, is displayed. Thus, a user using the medical image providing apparatus 100 feels as if the user diagnoses a fetus in the mother's womb while actually lighting the fetus with a flashlight.

The image that is provided by the medical image providing apparatus 100 may induce a user's concentration on a region of interest by displaying the region of interest brighter than a region other than the region of interest. In addition, the medical image providing apparatus 100 may more clearly display flections on a region of interest, e.g., a fetal wrinkle, the number of fingers, and the like, by adjusting the brightness of the region of interest and the extent of a shadow spread in an image that is generated by the medical image providing apparatus 100, and thus may improve a diagnosis accuracy of a user.

As illustrated in FIG. 5B, the display unit 140 may further display a UI for receiving a user input related to characteristics of a light source, as well as display the generated 3D image. For example, the medical image providing apparatus 100 may determine a distance from an object to a light source and a radiation direction of the light source by receiving a user input that moves an arrow 502 relative to a sphere 501 illustrated in FIG. 5B.

FIGS. 6A through 6C are diagrams for explaining a method of generating a 3D image, according to an embodiment of the present invention.

The image generation unit 130 of FIG. 1 may generate a 3D image by performing a rendering on volume data. For example, a ray casting method may be used to render the volume data.

The image generation unit 130 may perform a ray casting for casting a virtual ray on the volume data with respect to a predetermined pixel included in the 3D image.

The image generation unit 130 may sample a plurality of points on the virtual ray. The plurality of points (hereinafter, referred to as sampling points) may be positioned between voxels. Thus, it is necessary to interpolate values of samples from the voxels surrounding the sampling points. The gradient of an illumination value is calculated for each sampling point. The gradient indicates an orientation of a local surface in the volume data.

Next, the image generation unit 130 shades the sampling points according to the orientation of the local surface and the position of a light source. That is, the image generation unit 130 determines the contrast and colors of the sampling points.

Finally, the image generation unit 130 determines final colors for pixels by arranging the shaded sampling points along the virtual ray. Such a compositing operation may be directly derived from a rendering formula.

As illustrated in FIG. 6A, the image generation unit 130 may generate a 3D image 601 by shading the sampling points on the virtual ray based on determined characteristics of a light source. Referring to the 3D image 601 illustrated in FIG. 6A, the image generation unit 130 may generate a 3D image focused on a region of interest (for example, fingers) that is included in an object (for example, a fetus).

In addition, as illustrated in FIG. 6B, the image generation unit 130 may generate a 3D image 602 by shading sampling points on a virtual ray based on characteristics of a directional light passing through a predetermined filter. The filter is set to correspond to determined characteristics of a light source. That is, the filter may be set so that the directional light passing through the filter has the determined characteristics of the light source.

The filter is set differently according to a size and structure of an object. The filter may be used to adjust the brightness of a region of interest or a region other than the region of interest. In addition, for an accurate diagnosis, it is helpful to greatly increase the brightness of a region of interest appearing in a 3D image while decreasing the brightness of a region other than the region of interest by using the filter.

For example, the filter may be located spaced apart from an object by a distance corresponding to a determined distance from the object to a light source. Alternatively, the filter may be located at a position and angle, which corresponds to a determined radiation direction of the light source.

Referring to the 3D image 602 illustrated in FIG. 6B, the image generation unit 130 may generate a 3D image focused on a region of interest (for example, fingers) that is included in an object (for example, a fetus).

As illustrated in FIG. 6C, the image generation unit 130 may generate a directional light image 603 by shading sampling points on a virtual ray based on characteristics of a directional light. The image generation unit 130 may generate a 3D image 604 by processing the directional light image 603 based on determined characteristics of a light source. Referring to the 3D image 604 illustrated in FIG. 6C, the image generation unit 130 may generate a 3D image focused on a region of interest (for example, fingers) that is included in an object (for example, a fetus).

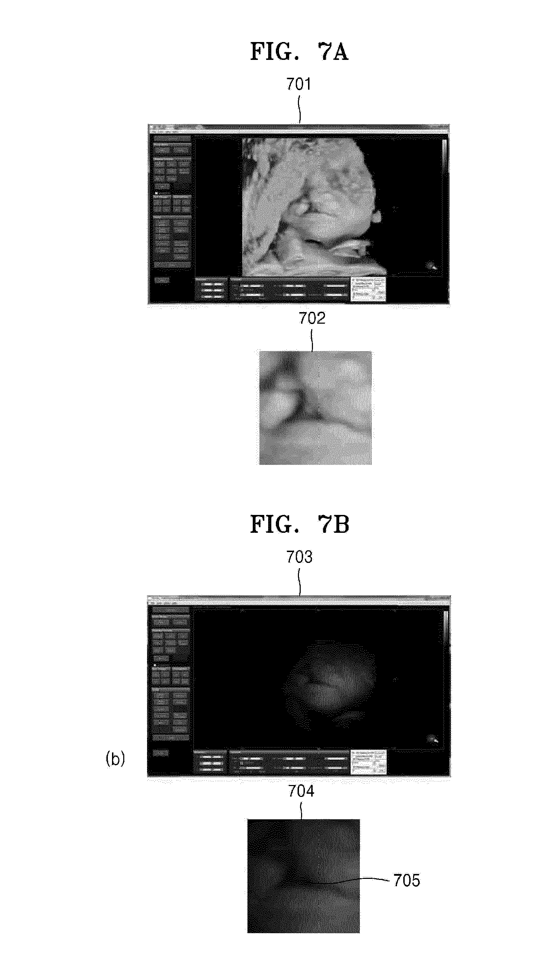

FIG. 7A illustrates 3D images 701 and 702 that are displayed according to an FRV. The 3D images 701 and 702 of a fetus, which are displayed according to the FRV, are 3D images generated based on a directional light.

FIG. 7B illustrates 3D images 703 and 704 that are displayed in the medical image providing apparatus 100.

Compared to the 3D images 701 and 702 illustrated in FIG. 7A, the 3D images 703 and 704 illustrated in FIG. 7B, which are provided by the medical image providing apparatus 100, are useful for a diagnosis of a fetus. A method of diagnosing a cleft lip 705 of a fetus by using the medical image providing apparatus 100 is described below with reference to FIG. 7B.

The medical image providing apparatus 100 may allow a user to concentrate more on a region of interest included in an object by displaying the region of interest, for example, the lips, fingers, or wrinkles of a fetus to be diagnosed, brighter than a region other than the region of interest.

In addition, the medical image providing apparatus 100 may provide a more realistic and detailed 3D image of a region of interest of an object by adjusting the brightness of the object or the extent of a shadow spread in the 3D image based on a user input. That is, a user that is provided with a 3D image from the medical image providing apparatus 100 may feel as if the user diagnoses a fetus while lightening the fetus in a mother's womb with a flashlight.

A medical image providing apparatus according to another embodiment of the present invention may further display a directional light image (for example, the 3D images 701 and 702 of FIG. 7A) generated by performing a rendering on volume data based on characteristics of a directional light. Based on a user input, the medical image providing apparatus 100 may replace the displayed directional light image with a determined light source-based 3D image (for example, the 3D images 703 and 704 of FIG. 7B) and then may display the 3D image.

That is, when an external input for instructing the medical image providing apparatus 100 to replace a directional light image with an image generated based on a determined light source (for example, a spotlight image) and to display the replaced image is received while the medical image providing apparatus 100 displays the directional light image, the medical image providing apparatus 100 may display the spotlight image corresponding to the directional light image. When an external input for instructing the medical image providing apparatus 100 to replace a spotlight image with a directional light image and to display the replaced image is received while the medical image providing apparatus 100 displays the spotlight image, the medical image providing apparatus 100 may display the directional light image corresponding to the spotlight image.

The invention can also be embodied as computer-readable codes on a computer-readable recording medium. The computer-readable recording medium is any data storage device that can store data which can be thereafter read by a computer system. Examples of the computer-readable recording medium include read-only memory (ROM), random-access memory (RAM), CD-ROMs, magnetic tapes, floppy disks, optical data storage devices, etc. The computer-readable recording medium can also be distributed over network-coupled computer systems so that the computer-readable code is stored and executed in a distributed fashion. Also, functional programs, codes, and code segments for accomplishing the present invention can be easily construed by programmers skilled in the art to which the present invention pertains.

As described above, the method and apparatus for providing medical images, according to the embodiments of the present invention, may improve a user's concentration on an image and a diagnosis accuracy of a user by providing a more realistic and detailed 3D image of a region of interest of an object.

While this invention has been particularly shown and described with reference to exemplary embodiments thereof, it will be understood by those skilled in the art that various changes in form and details may be made therein without departing from the spirit and scope of the invention as defined by the appended claims.

## Claims

1. A method of providing a medical image from volume data for an object, the method comprising:
determining characteristics of a light source that radiates light onto the object which is located in a virtual space, based on a received user input;
generating a three-dimensional (3D) image by performing a rendering on the volume data, based on the determined characteristics of the light source; and
displaying the generated 3D image.

2. The method of claim 1, wherein the characteristics of the light source comprise at least one of a distance from the object to the light source, a radiation angle of the light source, and a radiation direction of the light source.

3. The method of claim 1, wherein the determining of the characteristics of the light source comprises determining a distance from the object to the light source, and the generating of the 3D image comprises adjusting a brightness of at least a partial area of the 3D image based on the determined distance from the object to the light source.

4. The method of claim 1, wherein the determining of the characteristics of the light source comprises determining a radiation angle of the light source, and the generating of the 3D image comprises adjusting the extent of a shadow spread of at least a partial area of the 3D image based on the determined radiation angle of the light source.

5. The method of claim 1, wherein the determined characteristics of the light source comprise characteristics of a spot light or a point light.

6. The method of claim 1, wherein the generating of the 3D image comprises:
casting a virtual ray to the volume data;
shading sampling points on the virtual ray based on the determined characteristics of the light source; and
calculating a final color value of a predetermined pixel included in the 3D image from a cumulative value of shading values of the sampling points.

7. The method of claim 6, further comprising:
generating a directional light image by performing a rendering on the volume data based on characteristics of a directional light; and
displaying the generated directional light image,
wherein the displaying of the generated 3D image comprises replacing the directional light image with the 3D image and then displaying the 3D image, based on the user input.

8. The method of claim 1, wherein the generating of the 3D image comprises:
casting a virtual ray to the volume data;
shading sampling points on the virtual ray based on characteristics of a directional light passing through a predetermined filter; and
calculating a final color value of a predetermined pixel included in the 3D image from a cumulative value of shading values of the sampling points,
wherein the predetermined filter corresponds to the determined characteristics of the light source.

9. The method of claim 1, wherein the generating of the 3D image comprises:
generating a directional light image by performing a rendering on the volume data based on characteristics of a directional light; and
transforming the directional light image based on the determined characteristics of the light source.

10. The method of claim 1, wherein the determining of the characteristics of the light source comprises determining at least one of a shape, size, and gradation effect of at least a partial surface of the object that is brightly shown by radiating light thereto from the light source.

11. An apparatus for providing a medical image, the apparatus comprising:
a data acquisition unit that acquires volume data for an object;
a receiver that receives a user input;
an image generation unit that determines characteristics of a light source radiating light onto the object located in a virtual space based on the received user input and generates a 3D image by performing a rendering on the volume data based on the determined characteristics of the light source; and
a display unit that displays the generated 3D image.

12. The apparatus of claim 11, wherein the characteristics of the light source comprise at least one of a distance from the object to the light source, a radiation angle of the light source, and a radiation direction of the light source.

13. The apparatus of claim 11, wherein the determined characteristics of the light source comprise characteristics of a spot light or a point light.

14. The apparatus of claim 11, wherein the image generation unit determines the characteristics of the light source by determining at least one of a shape, size, and gradation effect of at least a partial surface of the object that is brightly shown by radiating light thereto from the light source.

15. A computer-readable recording medium having embodied thereon a computer program for executing the method of claim 1.
